(19) Europäisches Patentamt
European Patent Office
Office européen des brevets

(11) EP 3 378 388 A1

(12) **EUROPEAN PATENT APPLICATION**

(43) Date of publication:
26.09.2018 Bulletin 2018/39

(51) Int Cl.:
A61B 5/026 (2006.01)    A61B 5/00 (2006.01)

(21) Application number: 17162382.0

(22) Date of filing: 22.03.2017

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
MA MD

(71) Applicant: Cork Institute Of Technology
County Cork (IE)

(72) Inventors:
• Hegarty, Stephen P.
Cork City (IE)
• Gradkowski, Kamil
Cork (IE)

(74) Representative: Reedy, Orlaith
PurdyLucey
Intellectual Property
6-7 Harcourt Terrace
Dublin 2 (IE)

(54) **SYSTEM FOR MEASURING BLOOD FLOW VELOCITY**

(57) System and method for measuring blood flow velocity
The present invention provides a system and method for measuring the velocity of blood. The system comprises one or more modules configured to: generate a Doppler power spectrum from a Doppler shift signal detected from light reflected from a blood vessel; determine a linear region of the power spectrum; calculate the slope of the linear region of the power spectrum; and compare the calculated slope with calibration data to determine the velocity of blood in the blood vessel.

400 Detect the optical signal

405 Generate a time trace of the detected optical signal

410 Digitise the time trace by performing an analog to digital conversion

415 Perform a Fourier Transform on the digital signals to obtain a power spectrum

420 Select the linear region of the power spectrum

425 Fit a line to the linear region

430 Calculate the slope of the line

435 Determine the flow rate of the blood from a comparison of the slope with calibration data

Figure 4

## Description

## Field

The present invention is concerned with fluid flow rate measurements. More particularly, the invention is concerned with providing a system and a method for measuring the velocity or flow rate of blood.

## Background

Blood flow velocity measurements can provide hemodynamic information which may be used for a variety of purposes. For example, such information may be a useful aid in the diagnosis and in the treatment of blood vessel disease.

One known phenomenon which has previously been used to determine the velocity of blood is the Doppler effect. According to this phenomenon, when a wave is emitted or elastically scattered by an object that is traveling with a certain velocity, its frequency will be modified proportional to its velocity relative to the velocity of the observer. If the observers velocity is zero, then the only contribution to the frequency shift, called the Doppler shift $\Delta f$, comes from the object according to the equation set out below:

$$\Delta f = \frac{v}{c} f 0 = \frac{v}{c} \cdot \frac{c}{\lambda} = \frac{v}{\lambda}$$

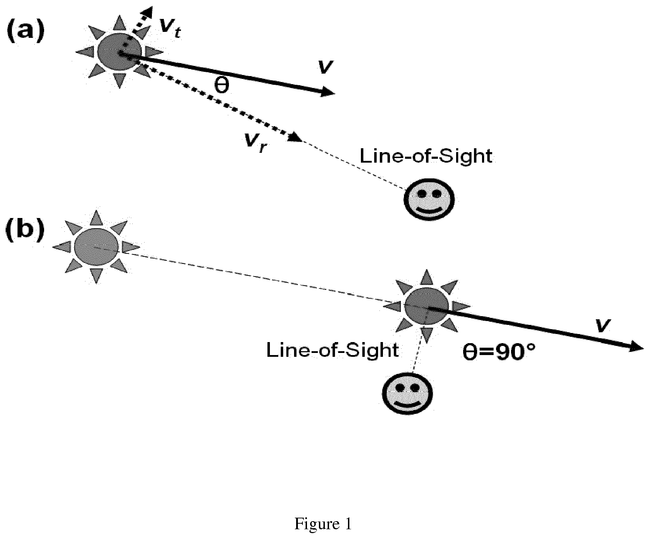

where $f0$ is the original frequency, $\lambda$ the wavelength, $c$ is the speed of wave in the medium (here the speed corresponds to the speed of light), and $v$ the velocity of the object. The magnitude of the shift will further be modified by the position of the observer relative to the object and its velocity vector, as illustrated in Figure 1. The observer in this case will experience an effective Doppler based on the radial component of the velocity vector $v$ which is its projection on the line-of-sight axis, as shown in Figure 1a, and is given by the equation below:

$$v_r = v \cos \theta$$

It will be appreciated that for lines of sight perpendicular to the velocity vector of the object, as shown in Figure 1b, the Doppler shift will be zero. Accordingly, it is not possible to measure any effect perpendicular to an object's motion, and its highest magnitude will be observed head-on.

Thus, by pointing monochromatic light at blood, and measuring at a detector the Doppler shift of the reflected or scattered wave according to the first equation set out above, it is in theory possible to determine the velocity of the blood. In the case of flowing blood, the measured shift spectrum (Doppler spectrum) corresponds to the sum of all the components of light scattered from the red blood cells traveling through the lumen of the blood vessel that return to the detector.

One known approach with regard to laser Doppler spectrum decomposition involves assuming a light scattering phase function (Henyey-Greenstein form), which has been shown to closely approximate the light scattering in blood. This function serves as a basis for the construction of a Doppler Scattering Density Probability (DSPD) matrix. The Doppler power spectra are decomposed by assuming a flow profile and fitting the matrix coefficients. This approach has been validated using Monte-Carlo simulations of photons interacting with an optically scattering medium. However, one drawback of this approach is that this technique requires a-priori knowledge of the anisotropy factor for light scattering, as well as an assumption about the flow profile. The technique is also quite complex to implement.

Accordingly, it is an object of the present invention to provide a system and method for obtaining blood flow velocity measurements which overcomes at least one of the above mentioned problems.

## Summary

According to the invention there is provided, as set out in the appended claims, a system for measuring the velocity of blood, the system comprising one or more modules configured to:

generate a Doppler power spectrum from a Doppler shift signal detected from light reflected from a blood vessel;
determine a linear region of the power spectrum;
calculate the slope of the linear region of the power spectrum; and
compare the calculated slope with calibration data to determine the velocity of blood in the blood vessel.

In an embodiment, the generation of a Doppler power spectrum from the Doppler shift signal comprises:

digitizing the Doppler shift signal; and
performing a Fourier Transform on the digitized signal.

In an embodiment, the determination of a linear region of the power spectrum comprises the selection of a range of frequencies for the linear region of the power spectrum.

In an embodiment, the selection of a range of frequencies for the linear region of the power spectrum comprises the selection of a predetermined range of frequencies for the linear region of the power spectrum.

In an embodiment, the selection of a range of frequencies for the linear region of the power spectrum comprises:

selecting an initial frequency as the lowest frequency in the frequency range;
determining the value of power corresponding to this initial frequency;
offsetting the determined power value by a threshold value to obtain a lower power value; and
determining the value of frequency corresponding to this lower power value as the highest frequency in the frequency range.

In an embodiment, the selection of a range of frequencies for the linear region of the power spectrum comprises:

selecting an initial frequency as the lowest frequency in the frequency range;
setting the lower power value to correspond to an offset above the noise floor associated with the power spectrum; and
determining the value of frequency corresponding to this lower power value as the highest frequency in the frequency range.

In an embodiment, the noise floor associated with the power spectrum was obtained during a calibration procedure.
In an embodiment, the calibration data comprises a calibration line constructed from a plot of a set of slopes as a function of inverted flow rate in respect of a plurality of known flow rates, each slope having been calculated from a line fitted to the linear region of a Doppler power spectrum associated with a particular flow rate.
In an embodiment, the comparison of the calculated slope with calibration data to determine the velocity of the blood in the blood vessel comprises:

determining from the calibration line the inverse flow rate value which is associated with the calculated slope; and
inverting the inverse flow rate value to determine the actual flow rate.

In an embodiment, the one or more modules are further configured to detect the voltage of a reflected light signal from a blood vessel over time, wherein the Doppler shift signal corresponds to the detected voltage signal.
In another aspect of the invention there is provided a method for measuring the velocity of blood the method comprising:

generating a Doppler power spectrum from a Doppler shift signal detected from light reflected from a blood vessel;
determining a linear region of the power spectrum;
calculating the slope of the linear region of the power spectrum; and
comparing the calculated slope with calibration data to determine the velocity of blood in the blood vessel.

In an embodiment, the step of generating of a Doppler power spectrum from the Doppler shift signal comprises:

digitizing the Doppler shift signal; and
performing a Fourier Transform on the digitized signal.

In an embodiment, the step of determining a linear region of the power spectrum comprises selecting a range of frequencies for the linear region of the power spectrum.
In an embodiment, the step of selecting a range of frequencies for the linear region of the power spectrum comprises selecting a predetermined range of frequencies for the linear region of the power spectrum.
In an embodiment, the step of selecting a range of frequencies for the linear region of the power spectrum comprises:

selecting an initial frequency as the lowest frequency in the frequency range;
determining the value of power corresponding to this initial frequency;
offsetting the determined power value by a threshold value to obtain a lower power value; and
determining the value of frequency corresponding to this lower power value as the highest frequency in the frequency range.

In an embodiment, the step of selecting a range of frequencies for the linear region of the power spectrum comprises:

selecting an initial frequency as the lowest frequency in the frequency range;
setting the lower power value to correspond to an offset above the noise floor associated with the power spectrum; and
determining the value of frequency corresponding to this lower power value as the highest frequency in the frequency range.

In an embodiment, the noise floor associated with the power spectrum was obtained during a calibration procedure.
In an embodiment, the calibration data comprises a calibration line constructed from a plot of a set of slopes as a function of inverted flow rate in respect of a plurality of known flow rates, each slope having been calculated from a line fitted to the linear region of a Doppler power spectrum associated with a particular flow rate.
In an embodiment, the step of comparing the calculated slope with calibration data to determine the velocity of the blood in the blood vessel comprises:

determining from the calibration line the inverse flow rate value which is associated with the calculated slope; and

inverting the inverse flow rate value to determine the actual flow rate.

The method may further comprise the step of:

detecting the voltage of a reflected light signal from a blood vessel over time,

wherein the Doppler shift signal corresponds to the detected voltage signal.

There is also provided a computer program comprising program instructions for causing a computer program to carry out the above method which may be embodied on a record medium, carrier signal or read-only memory.

## Brief Description of the Drawings

The invention will be more clearly understood from the following description of an embodiment thereof, given by way of example only, with reference to the accompanying drawings, in which:-

Figure 1 illustrates the geometry of the Doppler shift;
Figure 2 shows typical Doppler power spectra for a series of flow rates;
Figure 3 shows a schematic of one embodiment of the component set up in order for the system of the present invention to measure blood flow velocity;
Figure 4 shows a process flow of the main steps performed by the system of the present invention; and
Figure 5 shows two different embodiments for selecting the range of frequencies for the linear region of the Doppler power spectrum.

## Detailed Description of the Drawings

The present invention comprises a system configured to measure the velocity of flowing blood through the processing of Doppler spectra. The Doppler spectra may be obtained from the Doppler scattering of light from blood cells passing by the distal end of a guidewire positioned in a blood vessel in respect of which the velocity of blood flowing through the vessel is desired to be known. The guidewire incorporates an optical fibre configured to launch laser light into the blood vessel. However, unlike existing blood flow measurement techniques which use Doppler spectra to determine the velocity of blood, the present invention determines the velocity of blood based on an empirical analysis of the Doppler spectra, as will be explained in more detail below.

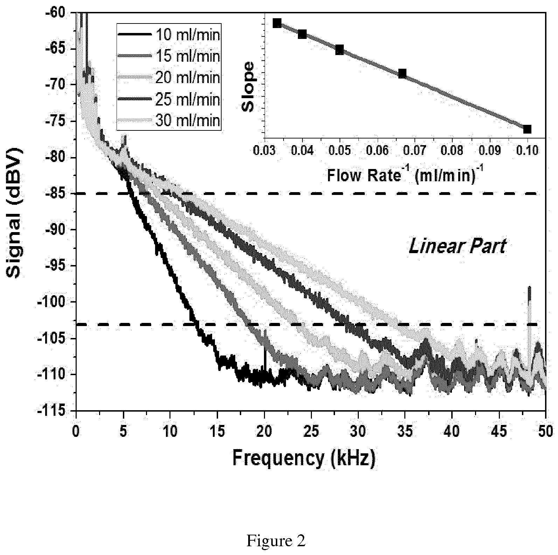

Figure 2 shows typical Doppler power spectra for a series of flow rates. It can be seen from this figure that a region of each spectrum is linear (as shown by the dashed horizontal lines). Accordingly, if a line is fitted to the linear portion of each spectrum, and the slope of this line is calculated, it is found that the slopes when taken as a function of inverted flow rate fall on a line, as illustrated in the inset of Figure 2. The present invention exploits this relationship between the Doppler power spectra and flow rate to measure the flow rate of blood.

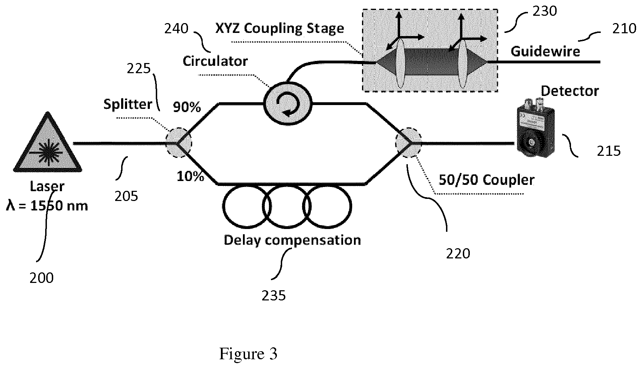

Figure 3 shows a schematic of one embodiment of the component set up in order for the system of the present invention to measure blood flow velocity.

An optical fibre 205 is coupled to a second optical fibre attached to a guidewire (GW) 210 for guiding light into a blood vessel (i.e. a guidewire fibre). A coupling stage 230 facilitates the optical coupling between the optical fibre 205 and the optical fibre 210. In one embodiment of the invention, the coupling is achieved through the use of two lenses.

An optical source 200 in the form of a laser provides the required light to the optical fibre 205. The operating wavelength of the laser is in the infra-red range. It is found that this range results in minimal absorption and a high scattering rate in blood. In one embodiment of the invention, the wavelength of the optical source 200 is set to 1550nm.

A detector 215 provides for the detection of the scattered laser light from the blood cells received at the guidewire fibre 210. A circulator 240 provides the necessary sequential coupling between the light emitted from the optical source 200 for transmission to the guidewire fibre 210, and the scattered light received at the guidewire fibre 210 to the detector 215.

In the described embodiment of the present invention, a technique known as optical heterodyne is used for detection. This involves splitting the light emitted from the optical source 200 into two paths at a splitter 225: a reference path, which passes the original light frequency $f_0$, and a probing (detection) path, which undergoes the Doppler shift in light frequency $\Delta f$. These two paths, when recombined by a coupler 220 and mixed in the detector 215 produce a beating signal of frequency $\Delta f$, which is the Doppler shift. The detection path carries 90% of the power, due to the fact that the scattering signal is very low. The reference path takes 10% of the power, since heterodyne does not require a large reference signal for conversion. The delay compensation fibre 235 ensures that the reference path length is very similar to the detection path length.

The guidewire fibre 210 should be positioned in the blood vessel in respect of which a blood flow measurement is to be determined. The laser 200 is then activated to emit light of the desired wavelength through the optical fibre 205 and into the blood vessel by means of the guidewire fibre 210.

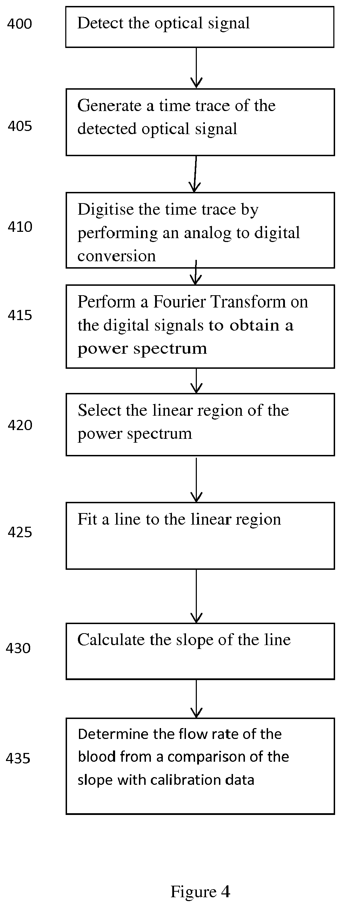

Figure 4 shows a process flow of the main steps performed by the system of the present invention in order to measure the velocity of blood in a blood vessel. In step 400, the optical signal received by the guidewire fibre 210 is detected by the detector 215. This optical signal corresponds to scattered laser light from blood cells in the blood vessel in which the guidewire fibre is positioned. The optical signal is processed to generate a time trace corresponding to the detected voltage of the optical signal over time (step 405). In step 410, the time trace is digitized by performing an analogue to digital conversion. In step 415, the digital signals are converted from the time domain to the frequency domain by means of a Fourier Transform, in order to obtain a Doppler power spectrum associated with the flow rate of the blood. In the case of a mix of frequencies coming from blood, this power spectrum will comprise a plurality of frequencies. The exact set of frequencies will depend on individual contributions of particle populations of the sampled blood.

In step 420, a range of frequencies for the linear region of the power spectrum is selected. In step 425, a line is fitted to this linear region. In step 430, the slope of the line is calculated. In step 435, the flow rate of the blood in the vessel is determined from a comparison of the slope with calibration data.

The calibration data corresponds to a calibration line which was previously constructed from a plot of a set of slopes as a function of inverted flow rate in respect of a plurality of known flow rates, each slope having been calculated from a line fitted to the linear region of a Doppler power spectrum associated with a particular flow rate. This process was previously explained in conjunction with Figure 2. In one embodiment of the invention, the calibration data may have been determined by connecting a linear pump to a lumen of appropriate diameter. This pump then is configured to deliver fluid flow at a set of selected flow rates. The Doppler spectrum corresponding to each flow rate is then recorded.

Thus, the comparison involves determining from the calibration line the value of the inverse flow rate which is associated with the calculated slope. The inverse flow rate value is then inverted to determine the actual flow rate of the blood.

The step of selecting the linear region of the resulting power spectrum can be implemented using a number of different techniques. Three different techniques for selecting the range of frequencies corresponding to the linear region of the power spectrum will now be described.

In one embodiment of the invention, a predetermined range of frequencies is selected for the linear region of the power spectrum. This approach is most suitable in the case where it is known a priori that the data will always be linear within this range.

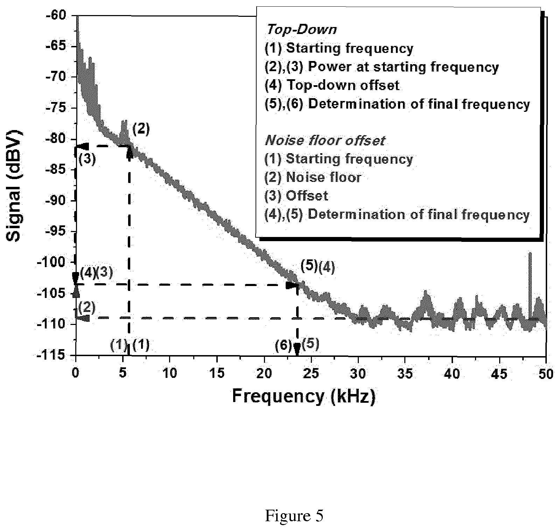

Figure 5 illustrates a second and a third embodiment for selecting the range of frequencies for the linear region of the power spectrum. In the second embodiment, labelled as "top down" in Figure 5, an initial frequency is selected as being the lowest frequency in the frequency range for the linear region of the power spectrum. The value of power corresponding to this initial frequency value is then determined. This power value is then offset by a predetermined threshold value to obtain the lower power value. Finally, the value of frequency corresponding to this lower power value is determined, which value corresponds to the highest frequency within the frequency range of the linear region of the power spectrum. This predetermined threshold value may be determined for example from experimental data values for the linear region of a power spectrum, and typically includes a margin for error.

In the third embodiment, labelled as "nose floor offset" in Figure 5, an initial frequency is again selected as the lowest frequency in the frequency range for the linear region of the power spectrum. The lower power value is then set to correspond to a certain offset above the position of the noise floor. The frequency corresponding to this lower power value is then determined, which value corresponds to the highest frequency within the frequency range of the linear region of the power spectrum.

The position of the noise floor may be determined during the process. Alternatively, the position of the noise floor may be recorded during an earlier calibration. It will be appreciated that as the noise floor is determined by the digitisation and laser noise, it is unlikely to vary significantly between experiments.

The advantage of the second and the third embodiment for selecting the range of frequencies corresponding to the linear region of the power spectrum over the first embodiment is that they offer increasingly higher potential detection ranges, due to the fact that they become increasingly independent from pre-determined frequency values.

It will be appreciated that any other suitable technique for selecting the range of frequencies for the linear region of the power spectrum could equally well be used, such as for example numerical averaging of the spectrum.

In one embodiment of the invention, the Fourier Transform of the digital signals is calculated by LabVIEW software running on a processor performing a Fast Fourier Transform. However, it should be appreciated that the Fourier Transform may be calculated through any suitable means.

It will be understood that the sampling rate of the analogue to digital conversion must be sufficient to encompass the range of Doppler-shifted frequencies, which is twice the expected maximum frequency in accordance with the Nyquist theorem.

It will be appreciated that the accuracy of the present invention is dependent on the length of the Fourier transform and the number of acquisitions (averages). In this regard, it will be appreciated that if the flow rate under study is substantially periodic, by acquiring the data multiple times and taking the average flow rate of all the included periods, the resulting measurement will be less noisy than any single measurement. Furthermore, it is known that flow recovery noise can increase for higher flow rates, as a result of the inverse relationship between velocity and slope. In order to reduce this effect, the acquisition parameters and calibration may be tailored to minimise the noise in the region of interest.

The present invention provides numerous advantages over existing techniques for measuring blood flow rate. One such advantage is the reduced computation complexity when compared to existing methods, which require significant computational effort. In addition, the present invention can operate in real time.

In the specification the terms "comprise, comprises, comprised and comprising" or any variation thereof and the terms include, includes, included and including" or any variation thereof are considered to be totally interchangeable and they should all be afforded the widest possible interpretation and vice versa.

The invention is not limited to the embodiments hereinbefore described but may be varied in both construction and detail.

## Claims

1. A system for measuring the velocity of blood, the system comprising one or more modules configured to:

   generate a Doppler power spectrum from a Doppler shift signal detected from light reflected from a blood vessel;
   determine a linear region of the power spectrum;
   calculate the slope of the linear region of the power spectrum; and
   compare the calculated slope with calibration data to determine the velocity of blood in the blood vessel.

2. The system of claim 1, wherein the generation of a Doppler power spectrum from the Doppler shift signal comprises:

   digitizing the Doppler shift signal; and
   performing a Fourier Transform on the digitized signal.

3. The system of any of the preceding claims, wherein the determination of a linear region of the power spectrum comprises the selection of a range of frequencies for the linear region of the power spectrum.

4. The system of Claim 3, wherein the selection of a range of frequencies for the linear region of the power spectrum comprises the selection of a predetermined range of frequencies for the linear region of the power spectrum.

5. The system of Claim 3, wherein the selection of a range of frequencies for the linear region of the power spectrum comprises:

   selecting an initial frequency as the lowest frequency in the frequency range;
   determining the value of power corresponding to this initial frequency;
   offsetting the determined power value by a threshold value to obtain a lower power value; and
   determining the value of frequency corresponding to this lower power value as the highest frequency in the frequency range.

6. The system of Claim 3, wherein the selection of a range of frequencies for the linear region of the power spectrum comprises:

   selecting an initial frequency as the lowest frequency in the frequency range;
   setting the lower power value to correspond to an offset above the noise floor associated with the power spectrum; and
   determining the value of frequency corresponding to this lower power value as the highest frequency in the frequency range.

7. The system of Claim 6, wherein the noise floor associated with the power spectrum was obtained during a calibration procedure.

8. The system of any of the preceding claims, wherein the calibration data comprises a calibration line constructed from a plot of a set of slopes as a function of inverted flow rate in respect of a plurality of known flow rates, each slope having been calculated from a line fitted to the linear region of a Doppler power spectrum associated with a particular flow rate.

9. The system of Claim 8, wherein the comparison of the calculated slope with calibration data to determine the velocity of the blood in the blood vessel comprises:

   determining from the calibration line the inverse flow rate value which is associated with the calculated slope; and
   inverting the inverse flow rate value to determine the actual flow rate.

10. The system of any of the preceding claims, where one or more of the modules are further configured to:

    detect the voltage of a reflected light signal from a blood vessel over time,
    wherein the Doppler shift signal corresponds to the detected voltage signal.

11. A method for measuring the velocity of blood the method comprising:

generating a Doppler power spectrum from a Doppler shift signal detected from light reflected from a blood vessel;

determining a linear region of the power spectrum;

calculating the slope of the linear region of the power spectrum; and

comparing the calculated slope with calibration data to determine the velocity of blood in the blood vessel.

12. A computer program comprising program instructions for causing a computer to perform the method of Claim 11.

Figure 1

Figure 2

Figure 3

400     Detect the optical signal

405     Generate a time trace of the detected optical signal

410     Digitise the time trace by performing an analog to digital conversion

415     Perform a Fourier Transform on the digital signals to obtain a power spectrum

420     Select the linear region of the power spectrum

425     Fit a line to the linear region

430     Calculate the slope of the line

435     Determine the flow rate of the blood from a comparison of the slope with calibration data

Figure 4

Figure 5

Europäisches
Patentamt

European
Patent Office

Office européen
des brevets

**EUROPEAN SEARCH REPORT**

Application Number

EP 17 16 2382

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 5 598 841 A (TANIJI AYAFUMI [JP] ET AL) 4 February 1997 (1997-02-04)<br>* column 1, lines 7-12 *<br>* column 2, line 58 - column 3, line 7 *<br>* column 3, lines 55-57 *<br>* column 4, lines 56-61 *<br>* column 5, lines 25-26,54-63 *<br>* column 7, lines 44-55 *<br>* figures 1,2,6A *<br>----- | 1-12 | INV.<br>A61B5/026<br>A61B5/00 |
| T | GERT E NILSSON ET AL: "Evaluation of a Laser Doppler Flowmeter for Measurement of Tissue Blood Flow",<br>IEEE TRANSACTIONS ON BIOMEDICAL ENGINEERING, IEEE SERVICE CENTER, PISCATAWAY, NJ, USA,<br>vol. BME-19, no. 10,<br>1 October 1980 (1980-10-01), pages 597-604, XP011174513,<br>ISSN: 0018-9294<br>* the whole document *<br>----- | 1-12 | TECHNICAL FIELDS SEARCHED (IPC)<br><br>A61B |

The present search report has been drawn up for all claims

| Place of search | Date of completion of the search | Examiner |
|---|---|---|
| Munich | 16 October 2017 | Willig, Hendrik |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

EP 3 378 388 A1

## ANNEX TO THE EUROPEAN SEARCH REPORT
## ON EUROPEAN PATENT APPLICATION NO.

EP 17 16 2382

This annex lists the patent family members relating to the patent documents cited in the above-mentioned European search report.
The members are as contained in the European Patent Office EDP file on
The European Patent Office is in no way liable for these particulars which are merely given for the purpose of information.

16-10-2017

| Patent document cited in search report | | Publication date | Patent family member(s) | | Publication date |
|---|---|---|---|---|---|
| US 5598841 | A | 04-02-1997 | JP | 3313841 B2 | 12-08-2002 |
| | | | JP | H0792184 A | 07-04-1995 |
| | | | US | 5598841 A | 04-02-1997 |

EPO FORM P0459

For more details about this annex : see Official Journal of the European Patent Office, No. 12/82